# EUROPEAN PATENT APPLICATION

(11) **EP 2 425 866 A1**
(43) Date of publication of application: **07.03.2012**
(21) Application number: 10008151.2
(22) Date of filing: 04.08.2010
(51) Int. Cl.: A61L 27/34, A61L 29/08, A61L 31/10

(54) **Multifunctional coating with antimicrobial activity and cell adhesion regulating surface characteristics as well as a method for preparing the same**

(71) Applicant: Max-Planck-Gesellschaft zur Förderung der Wissenschaften e.V., 80539 München (DE)
(72) Inventor: Förch, Renate, 55127 Mainz (DE); Duque, Luis, 55118 Mainzd (DE); Lotz, Alexander, 55118 Mainzd (DE)
(74) Representative: Katzameyer, Michael

(57) **Abstract**

The present invention provides a multifunctional coating having antimicrobial activity and the capability to regulate cell adhesion, comprising, deposited on a substrate surface,
i) an optional anchoring layer,
ii) a Zn-release layer deposited on the anchoring layer or directly on the substrate surface and
iii) a functional barrier layer deposited on the Zn-release layer, wherein the functional barrier layer is capable to slow down the diffusion of Zn from the Zn-release layer through the barrier layer and wherein the surface of the functional barrier layer regulates cell adhesion,
as well as a method for preparing said multifunctional coating.

In a more specific embodiment of the invention, the polymeric film of the Zn-release layer has been formed by plasma assisted polymerisation of zinc-organic precursors, e.g. Zn(acac)₂, bis(pentafluoro-phenyl)-zinc, and zinc-hexafluoro acetylacetonate.

## Description

The most common cause of hospital acquired infection today is the entry of bacteria such as Staphylococcus aureus into fresh wounds during an operation or during the first 3-4 days following an operation and before the wound has closed thus offering no further pathways of entry. Infections occur as a result of transfer of airborne and surface attached spores (on surgical equipment and/or the implant itself) and bacteria into the wound.

Materials such as steel, Teflon, and titanium and their composites are generally used for implantations since they have been proven to be (a) biocompatible and (b) "slow" fouling. While this "slow fouling" ability is advantageous for reducing bacterial infection, at the same time it prevents the incorporation of long lived implants into the tissue (e.g. dental implants, cardiovascular stents) thus enhancing rejection of the implant into the body.

Therefore, it is very desirable to improve the surface characteristics of such implant materials in order to control the adhesion of cells from the host tissue thus facilitating and promoting integration of implants into the host body. In this case either a cell adhesion promoting top layer, or a cell adhesion reducing top layer are desirable depending on the application or location of the implant in the body. For this purpose, several methods have been proposed and a common approach involves the deposition of an organic polymer film on the substrate surface. A number of polymers, such as polyallylamine or pentafluorophenyl methacrylate, are known to enhance cell adhesion properties. Plasma polymerised polyethylene glycol films and related materials have been shown to exhibit reduced cell adhesion properties. Both types of layers can be provided on implant surfaces by means of e.g. plasma assisted deposition. Moreover, even the "slow fouling" properties of the implant materials mentioned above are not fully satisfying and an enhancement of the antimicrobial properties of such implant materials is desirable.

In an attempt to simultaneously address the problem of both antimicrobial activity and improved cell adhesion, Schröder et al. (Materials Science Forum Vols. 638-642 (2010), 700-705) developed a titanium-based implant material wherein copper, which is a known antimicrobial material, has been introduced into the Ti implant by means of ion implantation techniques. Subsequently, the Ti surface has been coated with a polyallylamine film by means of plasma assisted deposition/polymerisation in order to improve the cell adhesion characteristics of the implant surface as well. Said approach suffers from the drawbacks that additional equipment as well as laborious and time consuming additional process steps are required for the incorporation of the antimicrobial copper dopant into the Ti material itself. Also, these steps involve rather high temperatures and are only applicable to metallic implants.

In US 2009/0187258 A1 medical implants which are able to induce fibrosis within the patient's body are described and as one suitable implant material among others zinc or a zinc alloy is disclosed. The implants can be surface-modified, e.g. for improving bioresorption. None of the examples actually uses zinc as an implant material and there is no specific disclosure with respect to this option.

In view of this prior art, an object of the present invention is to provide an improved multifunctional coating with excellent antimicrobial activity and cell adhesion regulating, in particular cell adhesion promoting or reducing, surface characteristics which can be produced in a fast, convenient and cost efficient manner. A related object of the invention is to provide an improved method for preparing such a multifunctional coating.

Said objects are solved by the coating of claim 1 and the method of claim 12. Further aspects and specific embodiments of the invention are the subject of additional claims.

The multifunctional coating having antimicrobial activity and the capability to regulate cell adhesion according to claim 1 comprises, deposited on a substrate surface,
i) an optional anchoring layer,
ii) a Zn-release layer deposited on the anchoring layer or directly on the substrate surface and
iii) a functional barrier layer deposited on the Zn-release layer, wherein the functional barrier layer is capable to slow down the diffusion of Zn from the Zn-release layer through the barrier layer and wherein the surface of the functional barrier layer regulates cell adhesion.

The substrate surface to be coated with the multifunctional coating of the present invention is not critical and may comprise or consist of a metallic, semi-metallic, polymeric, or ceramic material or any other material. Specifically, the substrate material is any biocompatible or bioresorbable material used for medical devices, in particular implants and surgical equipment. More specifically, the substrate is selected from the group of metals, in particular steel, Ti and their alloys and composites and organic polymers, e.g. Teflon, polyurethane, polyethylene, polypropylene.

The optional anchoring layer of the multifunctional coating according to the present invention may be any layer which is capable to bond to the respective substrate surface as well as to the Zn-release layer. It improves the stability of the multilayer on the substrate material, in particular in aqueous medium and in body fluids.

In one preferred embodiment, it consists of a plasma polymerised hexamethyldisiloxane film which can bond to an implant surface via Si-O bridges on the implant side and via Si-C- and Si-O bridges on the multilayer side. Techniques for preparing such an anchoring layer are known in the prior art (Cf.: Plasma polymerized hexamethyldisiloxane in adhesion applications, S. Pihan, T. Tzukruk, R. Förch, Surface & Coatings Technology 2009, 203, 1856-1862; Adhesion Improvement of plasma polymerized maleic anhydride films on gold using HMDSO/O2 adhesion layers, A.N. Chifen, A.T.A. Jenkins, W. Knoll, R. Förch, Plasma Processes & Polymers 2007, 4, 815-822; Recent and expected roles of plasma polymerized films for biomedical applications, Renate Förch, Anye N. Chifen, Angelique Bousquet, Hwei Ling Khor, Li-Qiang Chu, Zhihong Zhang, Ivy Osey-Mensah, Eva-Katrin Sinner, Wolfgang Knoll, Chemical Vapour Deposition 2007, 13, 280-294).

Alternatively, an anchoring layer can be formed by plasma treatment of the substrate surface with gases, e.g. argon, oxygen, nitrogen.

The antimicrobial activity of the present coating is based on the release of Zn (in the form of Zn²⁺ ions or Zn-clusters) from the Zn-release layer. Typically, said Zn-release layer comprises a Zn containing polymer coating deposited on the anchoring layer or directly on the implant surface, preferably by plasma assisted polymerisation. The use of zinc in the coating of the present invention is especially advantageous due to its well-known antimicrobial activity together with its ability to be metabolised by the human body and is thus considered to be of lower toxicity than, for example silver. This latter property gives zinc a particular advantage over other metals such as silver, which currently represents the state of the art and is present in a variety of commercial products in wound care, medical devices and implants. As demonstrated in the examples, the Zn in the polymeric film comprises nanoparticles of ZnO and/or Zn as well as complexed zinc ions. Typically, the nanoparticles have diameters in the range of 1 to 50 nm.

The polymeric Zn-release layer can be conveniently synthesised from organo-zinc monomers of low molecular weight that are suitable for plasma deposition processes. Preferably, the monomeric precursors are selected from the group of zinc-organic compounds, e.g. Zn(acac)₂ (zinc acetylacetonate), bis(pentafluorophenyl)-zinc, zinc-hexafluoro acetylacetonate and similar monomers. Typically, the Zn-release layer contains up to 10 atom-% Zn (XPS surface analysis) and has a thickness in the range of 1 to 500 nm.

The functional barrier layer is not in the traditional sense a barrier layer which prevents the diffusion of ions and molecules from one side to another. Instead, in the present sense of the word, the "barrier" coating merely slows down the diffusion of ions and molecules in order to regulate the release of an antimicrobial substance and at the same time offers an uppermost interface that controls or regulates cell adhesion.

The functional barrier layer may promote cell adhesion, either as such, due to its inherent surface characteristics, and/or by further immobilisation of cell adhesion motifs, e.g. fibrinogen, fibronectin, RGD.

Alternatively, the functional barrier layer may reduce the adhesion of cells without being cytotoxic due to the chemical nature of the surface.

This functional barrier layer or coating can be designed in different ways. Preferably, the present invention involves the deposition of a plasma polymerised functional thin layer from precursors such as allylamine (already well known to promote cell adhesion, refs. from Förch CVD Journal 2007 and others), maleic anhydride (refs. from Förch, Jenkins, and others, above) and pentafluorophenyl methacrylate (refs. from Förch & Borros, 2005, 2007, above). Plasma polymer layers deposited from these monomers are highly reactive thin films and can be further derivatised to attach adhesion peptides and proteins such as RGD or fibrinogen. It has been shown (Förch et al. unpublished) that such surface derivatised layers can further improve the adhesion of P19 progenitor cells as well as fibroblasts, endothelial cells and osteoblasts.

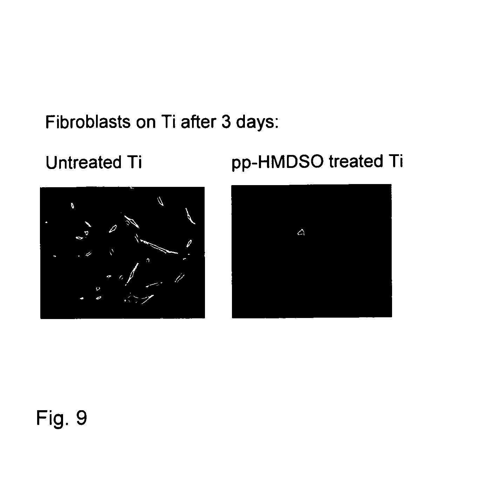

The inventors have demonstrated that plasma polymerised hexamethyldisiloxane exhibits reduced cell adhesion properties (Fig. 9) (see also refs. from Wei, J. Biomed. Mater. Research Part B: Applied Biomaterials, 2006 as well as Diploma theses of Lotz, MPIP Germany, 2009 and Kroth, MPIP Germany, 2009) and have further surprisingly found that this material can be advantageously used for providing a functional barrier layer suitable for the multifunctional coating of the invention which functional barrier layer additionally exhibits said antiadhesive surface characteristics.

Typically, the functional barrier layer has a thickness in the range of 1 to 500 nm.

The present invention relates also to a convenient and very effective method for producing the multifunctional coating as disclosed above.

A preferred method for producing the multifunctional coating of the invention comprises the steps:
a) providing an optional anchoring layer on a substrate surface, e.g. by plasma polymerisation or surface modification;
b) plasma deposition and simultaneous polymerisation of a Zn-containing organic monomer to provide a Zn-release layer;
c) plasma deposition and simultaneous polymerisation of one or more organic monomers capable to be polymerised into a polymer which regulates cell adhesion and provides a functional barrier layer.

More specifically, in the method for producing the multifunctional coating of the invention the optional anchoring layer is provided in step a) by polymerising, preferably plasma assisted polymerising, a siloxane compound, preferably hexamethyldisiloxane (HMDSO) or a structurally similar compound, or by plasma treatment of the substrate surface with gases, e.g. argon, oxygen, nitrogen.

Preferably, the Zn-containing organic monomers deposited and polymerised in step b) are selected from the group of Zn (acac) ₂, bis(pentafluorophenyl)-zinc, zinc-hexafluoro acetylacetonate or structurally related compounds.

In order to achieve further improvements of the properties of the Zn-release layer, the Zn containing organic monomers such as Zn (acac) ₂ may be mixed with other gases such as fluorocarbons, hydrogen, oxygen, argon, carbon dioxide, carbon monoxide. This allows for the modification of the polymer network structure and can be used to control or tune the diffusion of Zn within and out of the polymer matrix.

Typical, but non-limiting working conditions of the method for producing the multifunctional coating of the present invention are as follows: plasma powers in the range of 5 to 150 Watts, duty cycles for pulsed plasma conditions in the range of 1 to 50 %, working pressures in the range of 0,001 to 0.5 mbar.

Specifically, the organic monomers polymerised in step c) of the present method are selected from the group of allylamine, maleic anhydride, pentafluorophenyl methacrylate and hexamethyldisiloxane.

A further related aspect of the invention relates to a substrate, more specifically a medical device, e.g. an implant or surgical equipment, comprising the multifunctional coating as disclosed above.

The invention is illustrated in more detail by the following non-limiting examples and figures.

### FIGURES

**Fig. 1****.** schematic cross-sections of 2 variants of a multifunctional coating of the present invention
**Fig. 2****.** IRRAS spectra of pp-Zn (acac) ₂ films showing changes in chemical structure with increasing input powers
**Fig. 3****.** TEM micrographs of the same films demonstrating 2-4 nm sized ZnO/Zn nanoparticles within the films
**Fig. 4****.** Zn content of the films; (a) XPS elemental scans showing the presence of Zn in the Zn(acac)₂ plasma polymer; (b) summary of Zn content in the film as a function of position in reactor and input power
**Fig. 5****.** Kinetics of Zn release from a plasma polymerised Zn(acac)₂ film deposited
**Fig. 6****.** Metal release of an inventive multilayer consisting of the Zinc release system and plasma polmyerised allylamine of different thickness
**Fig. 7****.** Antimicrobial activity of plasma deposited Zn(acac)₂ films towards S.aureus and P. aruginosa; a) quantitative growth assay of bacteria attached, b) quantitative analysis of the bacteria in the surrounding medium
**Fig. 8****.** Cell adhesion of P19 progenitor cells on surface derivatised plasma polymerised PFM using different adhesion motifs, including the adhesion peptide IKVAV and the adhesion protein fibrinogen
**Fig. 9****.** Adhesion of fibroblast cells on a Ti substrate coated with plasma-polymerised hexamethyldisiloxane (pp-HMDSO) compared to the adhesion on an untreated Ti substrate

### Example 1

### Preparation of an antimicrobial and cell adhesion promoting coating

In a first step a polypropylene substrate surface was coated with an anchoring layer of a plasma polymerised hexamethyldisiloxane film which bonds to an implant surface via Si-O bridges on the implant side and via Si-C- and Si-O bridges on the multilayer side. It improves the stability of the multilayer on the implant material in aqueous medium.

Subsequently, plasma deposition of the different monomers in steps b) and c) of the inventive method was effected. Plasma deposition was carried out in a home-built capacitivly coupled cylindrical radio frequency (rf, 13.56 MHz) plasma reactor, 30 cm in length and 10 cm in diameter. (REF) The power was generated by an rf generator, and was coupled into the reactor chamber via two concentric, external ring copper electrodes placed 12 cm apart. The samples were placed within the chamber 1 cm from the leading hot electrode. Throughout the work the plasma was run under the continuous wave mode using an input power of 50 W. Because of the low volatility of the monomers used, the reactor inlet was equipped with a home-built heating jacket. The monomer supply vessel was heated close to the boiling point of the monomer using a thermal bath. The temperature of the glass reactor was monitored using a thermo sensor (PT-100) mounted on the arm inlet, and was controlled using a home built temperature controller. The reaction chamber was evacuated to 1x10⁻³ mbar using a rotary pump (Leybold Trivac, D16BCS PTPFE). A liquid nitrogen cold trap was used to collect excess vapour. A baratron (MKS, Type 627B) placed near the monomer inlet was used to monitor the pressure inside the chamber. The monomer pressure was typically 0.02 mbar, and the deposition time was adjusted to obtain the desired thickness.

Using the above process conditions a typical deposition rate of the Zn(acac)₂ monomer was 12 nm/min. For comparison and to validate the role of the ligand, the acetylacetonate ligand (acac) was also polymerised using the same process conditions. Some improvement over properties was achieved by mixing Zn(acac)₂ with other gases such as fluorocarbons or hydrogen. This allows for the modification of the polymer network structure and can possibly influence the diffusion of Zn within and out of the polymer matrix.

### Example 2

### Characterisation of the antimicrobial and cell adhesion promoting coating

### Chemical composition of the pp-Zn(acac)₂ films

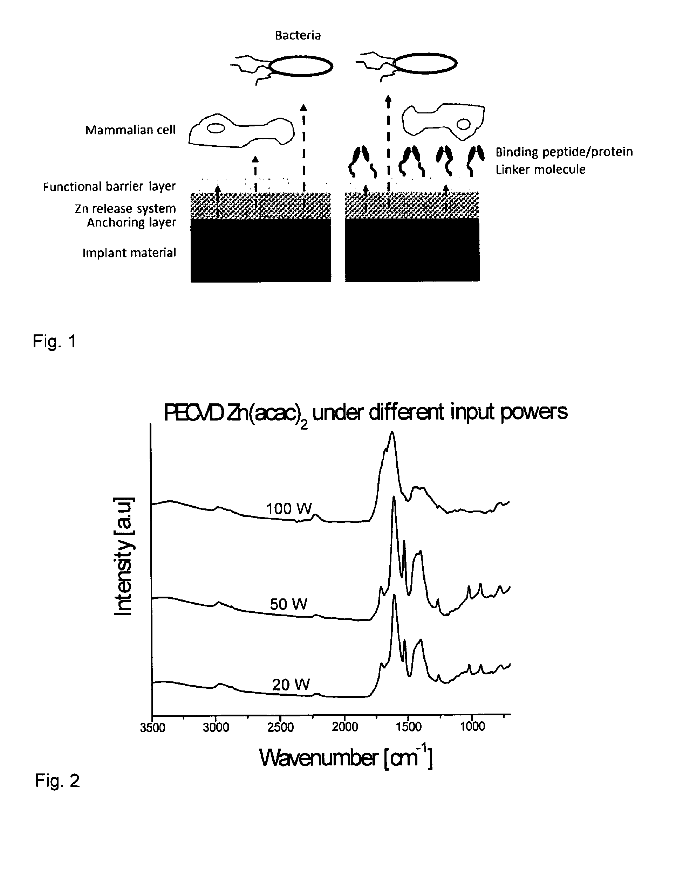

IRRAS spectra of Zn-doped a-C:O:H films are shown in Fig. 2, and show characteristics bands at ≈ 2950 cm⁻¹ and ≈ 1400 cm⁻¹ assigned to CH, CH₂ and CH₃. Other absorption bands at wavenumbers around 1710 cm⁻¹ are attributed to C=O bonds, probably coming from ∝, β unsaturated aldehydes and ketones. Furthermore, the absorption peak at 1600 cm⁻¹ is assigned to the stretching vibrational mode of C=C bond. Other oxide-related absorption peaks (range from 3400 to 3450 cm⁻¹) were weak and assigned to hydroxyl groups. Last, absorption bands at ≈ 1022 cm⁻¹ and ≈ 1260 cm⁻¹ correspond to other stretching modes from C-O groups, present in ester and ether groups. These results indicate that most of the oxygen atoms in the a-C:O:H film are bonded to hydrogen and carbon atoms. However, absorption intensity of C-Hₓ and C=C groups is much higher, suggesting that the oxygen is also bonded to another element; zinc.

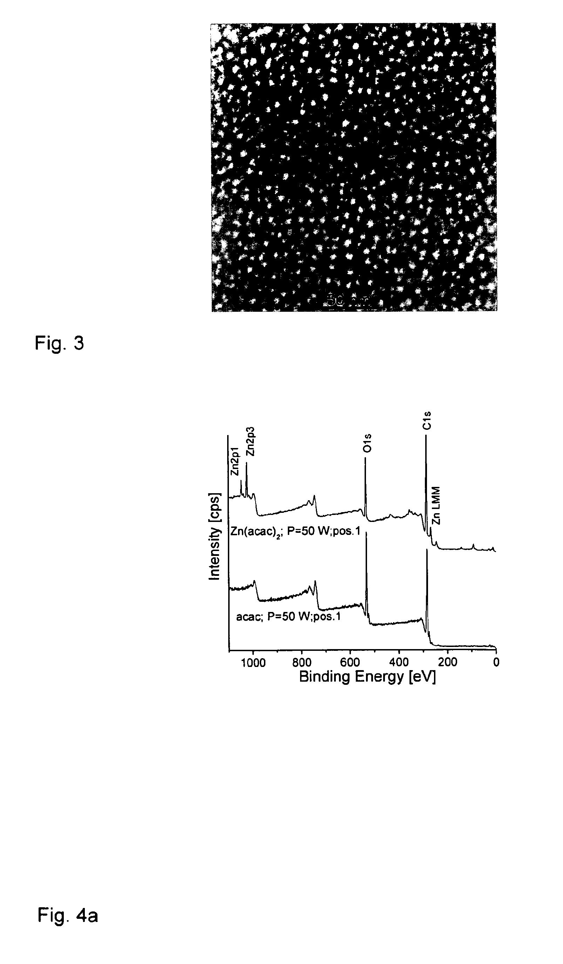

TEM micrographs of the same films showed 2-4 nm sized nanoparticles within the films (Fig. 3).

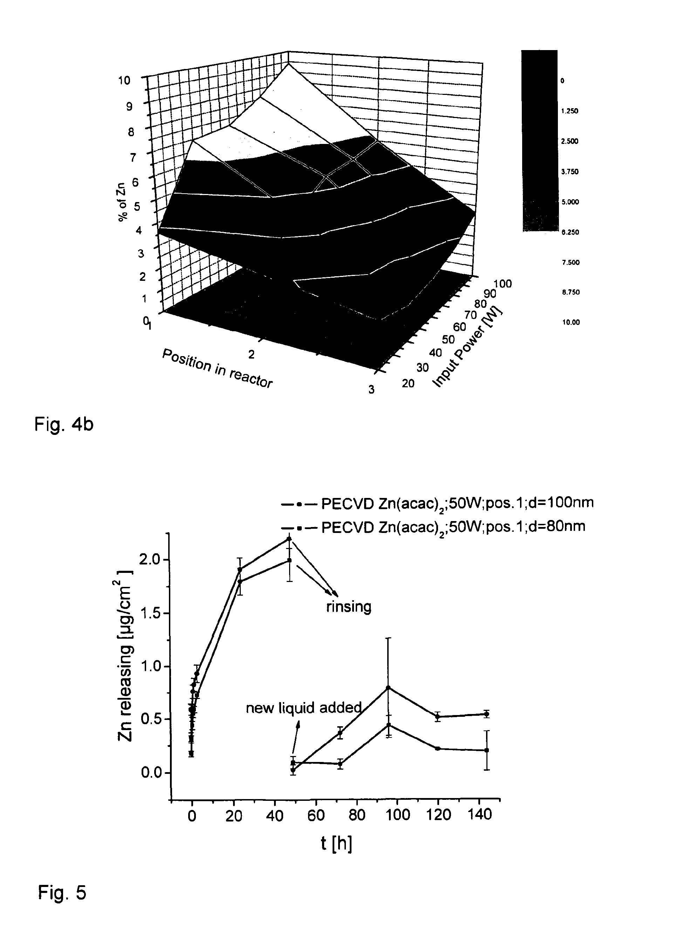

X-Ray Photoelectron Spectroscopy (Fig. 4) showed that the metal content can be varied and is dependent on the deposition conditions used. In the work to date 10 % Zn was the maximum. The Zn appears to be present within the film predominantly as small (2-4 nm) sized ZnO/Zn nanoparticles as shown by TEM (Fig. 3).

### Zinc release properties

Zinc release properties were analysed using Inductively Coupled Plasma-Optical Emission Spectroscopy (ICP-OES), (Fig. 5). It was observed that the Zn is released slowly over approximately 3-4 days. The rate of release is dependent on the process conditions used and thus the chemical structure of the surrounding polymer network. As shown in Fig. 5, at position 1 in the reactor the Zn release is very rapid and about 3/4 of the measured Zn is released within 3 days. On the other hand at position 2 within the reactor, which comprises a more reactive zone of the plasma, a higher crosslink density can be assumed within the polymer structure. It is observed that the overall Zn content, as well as the release of Zn is less such that only about 1/2 of the total Zn observed has been released within the same time frame.

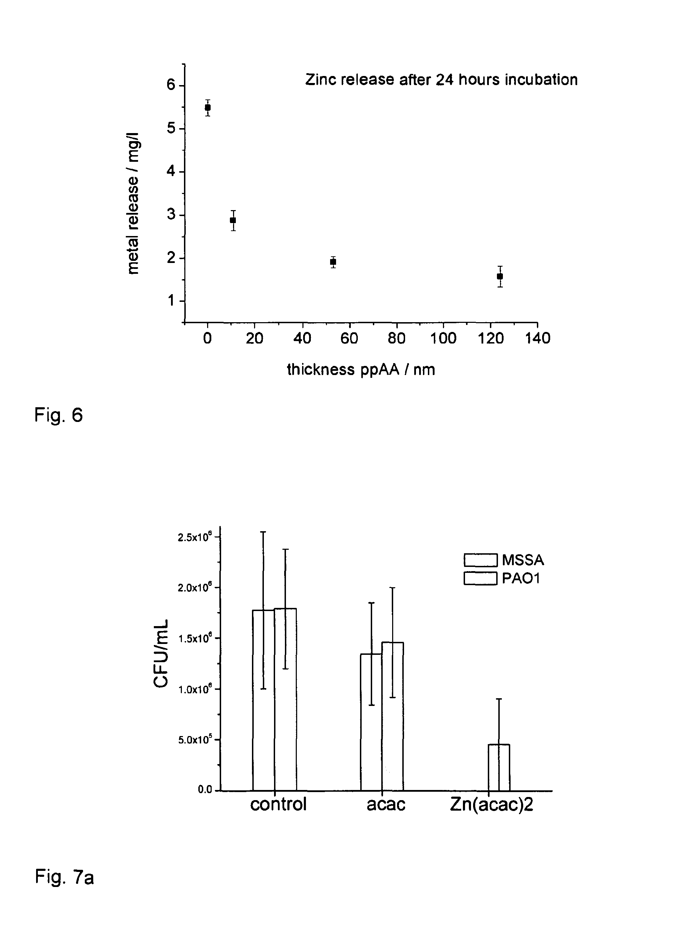

When depositing a plasma polymerised layer of allylamine, maleic anhydride, pentafluorophenyl methacrylate or related monomers on top of the zinc release system, the release of the zinc into the surrounding environment can be slowed down. Fig. 6 shows pp-allylamine films of different thickness deposited on top of the Zinc release system and the effect this has on the release of zinc from the multilayer system. Similar results are to be expected using other functional surface layers.

### Antimicrobial activity

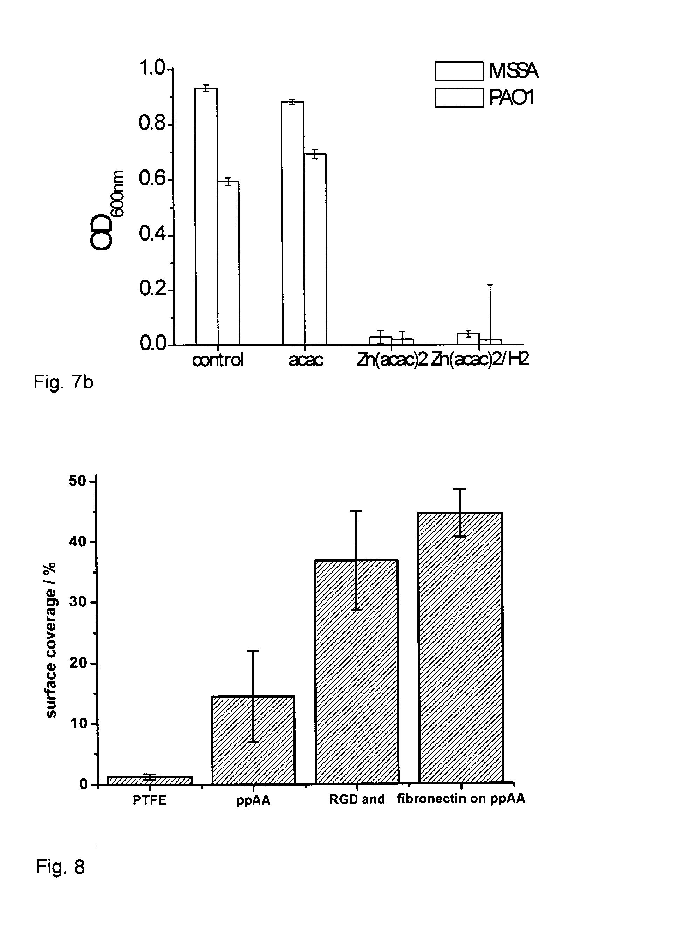

Antimicrobial activity was tested towards Staphylococcus aureus and Pseudomonas aruginosa using Zn(acac)₂ and Zn(acac)₂ mixed with H₂ during the deposition (Fig. 7). The plating results show a very clear trend and no growth of MRSA was observed for the Zn(acac)₂ films as only very low growth was observed for Zn(acac)₂ mixed with hydrogen.

Preliminary results show that a relatively low concentration of Zn released is effective in significantly reducing the attachment of S. aureus and *P. aeruginosa.*

## Claims

1. A multifunctional coating having antimicrobial activity and the capability to regulate cell adhesion, comprising, deposited on a substrate surface,
i) an optional anchoring layer,
ii) a Zn-release layer deposited on the anchoring layer or directly on the substrate surface and
iii) a functional barrier layer deposited on the Zn-release layer, wherein the functional barrier layer is capable to slow down the diffusion of Zn from the Zn-release layer through the barrier layer and wherein the surface of the functional barrier layer regulates cell adhesion.

2. The multifunctional coating according to claim 1, wherein the optional anchoring layer is produced by plasma polymerisation of hexamethyldisiloxane or plasma treatment of the substrate surface with gases, e.g. argon, oxygen, nitrogen.

3. The multifunctional coating according to claim 1 or 2, wherein the Zn-release layer is a polymeric film comprising nanoparticles of ZnO and/or Zn as well as complexed zinc ions.

4. The multifunctional coating according to any one of claims 1 to 3, wherein the polymeric film of the Zn-release layer has been formed by plasma assisted polymerisation of zinc-organic precursors, e.g. Zn(acac)₂, bis(pentafluorophenyl)-zinc, zinc-hexafluoro acetylacetonate.

5. The multifunctional coating according to any one of claims 1 to 4, wherein the functional barrier layer comprises an organic polymer which promotes cell adhesion.

6. The multifunctional coating according to any one of claims 1 to 4, wherein the functional barrier layer comprises a polymer which reduces cell adhesion.

7. The multifunctional coating according to claim 5 or 6, wherein the organic polymer of the functional barrier layer comprises a polymer film produced by plasma assisted polymerisation of monomers selected from the group consisting of allylamine, maleic anhydride, pentafluorophenyl methacrylate, hexamethyldisiloxane.

8. The multifunctional coating according to claim 5 or 7, wherein the organic polymer of the functional barrier layer comprises a polymer film produced by plasma assisted polymerisation of monomers and on which polymer film further cell adhesion molecules or motifs, e.g. fibrinogen, fibronectin, RGD, are immobilised.

9. The multifunctional coating according to any one of claims 1 to 8, wherein the Zn-release layer comprises ZnO/Zn nanoparticles having a diameter of 1 to 50 nm as well as complexed zinc ions.

10. A substrate comprising the multifunctional coating according to any one of claims 1 to 9.

11. A medical device, e.g. an implant or surgical equipment, comprising the multifunctional coating according to any one of claims 1 to 9.

12. A method for producing the multifunctional coating according to any one of claims 1 to 9, comprising the steps:
a) providing an optional anchoring layer on a substrate surface;
b) plasma deposition and simultaneous polymerisation of a Zn-containing organic monomer to provide a Zn-release layer;
c) plasma deposition and simultaneous polymerisation of one or more organic monomers capable to be polymerised into a polymer which regulates cell adhesion and provides a functional barrier layer.

13. The method of claim 12, wherein the anchoring layer is produced by plasma polymerisation of hexamethyldisiloxane or by plasma treatment of said substrate surface with gases.

14. The method according to claim 12 or 13, wherein the Zn-containing organic monomers in step b) are selected from the group of plasma polymerisable zinc-organic precursors, e.g. Zn(acac)₂, bis(pentafluorophenyl)-zinc, zinc-hexafluoro acetylacetonate.

15. The method according to any one of claims 12 to 14, wherein the organic monomers in step c) are selected from the group of allylamine, maleic anhydride, pentafluorophenyl methacrylate and hexamethyldisiloxane.

16. The method according to any one of claims 12 to 15, wherein in step b) the Zn-containing organic monomer is mixed with additional gases, e.g. fluorocarbons, hydrogen, oxygen, argon, carbon dioxide, carbon monoxide.
